# EUROPEAN PATENT APPLICATION

(11) **EP 3 150 112 A1**
(43) Date of publication of application: **05.04.2017**
(21) Application number: 15425076.5
(22) Date of filing: 30.09.2015
(51) Int. Cl.: A61B 5/00, A61B 5/053, A41B 11/00, A43B 3/00

(54) **AN IMPEDANCE MEASUREMENT FOOTWEAR SYSTEM**

(71) Applicant: Akern S.r.L., 50065 Pontassieve (FI) (IT)
(72) Inventor: Talluri, Antonio, 50012 Bagno a Ripoli (FI) (IT)
(74) Representative: Emmi, Mario

(57) **Abstract**

The present invention concerns an innovative footwear system, for example in the form of an insole or sock.

It comprises at least one injecting electrode (10', 10" , 100', 100"), through which to inject a current (I) of reference, and at least one receiving electrode (20', 20"; 200', 200") through which to measure a relative voltage drop (V) and/or a variation of phase.

## Description

### Technical field

The present invention concerns the technical field relative to the determination of one or more physiological parameters, in accordance with the tetra-polar technique.

In particular, the invention refers to an innovative footwear system, for example in the form of a sock or plantar, configured to allow to determine and monitor physiological parameters of the patient, such as the body hydration condition or the presence of foot diseases.

### Background art

The tetra-polar measurement technique of the body bioimpedance has long been known. It is known as a technique capable of executing quantitative and qualitative and/or diagnostic assessments of the body or of the bio-conducting tissues observed.

In general, the tetra-polar technique foresees the use of a first pair of electrodes, connected to a generator (G). Such electrodes are capable of injecting a first alternate current in the subject to which they are applied.

A second pair of electrodes is then foreseen that is generally connected to a voltmeter (V) which measures the voltage drop at the ends of said two electrodes so that a processor can derive the impedance Z and from here a series of physiological measurements with known formulas, among which the hydration condition.

Together with the voltmeter (V) methods of measurement of the dephasement of the injected current (impedentiometric systems sensible to the phase) can also be used.

In accordance with the known tetra-polar technique, the generator (G) generates an alternate current, kept constant or of a known constancy, and signal, preferably sinusoidal with frequencies that vary from 1 kHz to 1000 kHz. The most diffused frequency is 50 kHz. The values of the constant current that are the most diffused and used in accordance with the present invention range between 100 and 800 micro amperes.

Having said that, various devices of the portable type are currently known that allow to determine various physiological parameters of a subject, among which also the body hydration condition, and some of such devices are even aimed at a strictly sporting use.

For example, impedentiometric scales with electrodes for the injection of current and detection of voltage drop to the soles are known and, through the detection also of the weight, their processor is programmed to derive values of total water, fat mass and thin mass with formulas that are well-known and tested, which therefore give a quite reliable result.

Nevertheless, such impedentiometric scales limit themselves to a derivation of physiological parameters that are strictly correlated with the weight and, above all, are static devices, in the sense that the user rises on them on bare feet and makes an instantaneous measurement, remaining still on the scales for a couple of seconds. It is therefore not possible through them to make measurements of parameters during a period of motor activity of the subject, limiting the observation as a consequence. They are therefore not idoneous to execute a continuous monitoring or with a mid-long term data sampling.

Other wearable impedentiometric devices are known to make measurements in other parts of the human body (for example pulse and chest) but these are of such a small size as to oblige the use of extremely small electrodes which, if on the one hand have the advantage of not being of encumbrance, on the other hand they offer scarce precision and repetitiveness.

Further, the conformation of such devices is not such as to guarantee always a contact of the electrodes that is precise and constant with the skin.

A case is, for example, relative to the bracelet that is currently in commerce and described on the following Internet address:

### http://www.bbc.com/news/technology-30681002

The bracelet/watch is therefore (as per tetra-polar standard impedentiometry) capable of injecting a current through two electrodes and detecting a voltage drop so as to obtain bio-impedentiometric values.

Such a bracelet/watch is particularly useful and has been studied precisely for sporting use, since a monitoring of the variations of the hydration condition is fundamental to complete in the best way a sporting performance (both a training session and a competition).

Nevertheless, due to the configuration and conformation it is difficult to always guarantee a contact of the electrodes if not tightening the bracelet around the pulse and therefore creating, in fact, a circulation problem that could disturb the measurement. Further, the reduced dimensions often alter in fault the measurement detected. Last, the measurement made has the disadvantage of being acquired with an arm that can be positioned in different ways: upright in vertical position, laid in horizontal position, or beside the hips.

Each one of these positions induces a significant variation of the water volume in the tissues (due to the earth gravity) with a consequent significant variation of electric conductivity, rendering impossible or anyway difficult to distinguish signal from artefact.

### Disclosure of invention

It is therefore the aim of the present invention to provide an innovative system capable of detecting one or more physiological parameters of a subject, which solves said technical inconveniences.

In particular, it is the aim of the present invention to provide a system that affords the need to obtain physiological measurements of the foot or of the inferior hemibody in a precise and repetitive way.

In particular, it is the aim of the present invention to provide a system that, in a precise and comfortable way for the user and with a highly repetitive positioning, allows to detect one or more physiological parameters, among which the body hydration condition, the body composition or even the eventual presence of injuries of the foot, both for medical and sporting use.

It is also the aim of the present invention to provide a system that allows the precise detection of such physiological parameters both during a period of time in which the user is free to move in any motor activity, also a sporting one, and also in case of immobility.

These and other aims are therefore reached with a footwear system 1, as per claim 1.

Such a footwear system is obviously configured to result in use in contact with at least one part of the foot, in particular the sole.

It foresees at least one injecting electrode (10', 10", 100', 100"), through which to inject a current (I) of reference, and at least a receiving electrode (20', 20", 200', 200") through which to measure a relative voltage drop (V) and/or a variation of phase.

Through the injection of current and the measurement of the relative voltage drop various physiological parameters, also parameters with reference to the foot, as it is well known, can be measured.

The footwear system is easily worn by the user, who can thus be monitored easily during its motor activity or in case he is immobilized on the bed.

The footwear system, in general, allows the insertion of electrodes of big size that guarantee a good contact and consequently a precise measurement, above all if used upright during the motor activity. The body weight of the subject, in fact, guarantees a good contact between skin and electrode, the whole being an advantage for and helping the precision of the measurement made.

In a possible solution, the footwear system can, for example, be in the form of an insole or pair of insoles and, in a variant, in the form of a sock or pair of socks.

Two different insoles (or a pair) can therefore be used, having, each one, a single injecting electrode and a single receiving electrode and connecting said soles between them through generator and voltmeter that are positioned externally to the insole.

This solution allows to monitor the inferior hemibody.

Alternatively, a single insole can be used having a pair of injecting electrodes and a pair of receiving electrodes or, in a preferred alternative, a pair of such insoles if both feet are to be monitored.

In this case, the foot area is very well monitored and it is possible to integrate the voltmeter and the generator in the body of the insole.

In the case of insoles, these can be used with sock realized in part in a conducting yarn that overlaps to the electrodes of the insoles to function as transmission interface. In this way, the user can use the socks, generally necessary in case of motor activity.

In a variant of the invention, such a system can be, *di per se*, only in the form of a sock or a pair of socks. This solution is useful for people immobilized on the bed that have to be monitored without creating disturbs and, on the contrary, allowing the use of a protection that generally is worn always, above all in the winter period.

In that case, the sock is realized in part in conducting yarn so as to obtain at least two electrodes (for measurements of hemibodies), or four electrodes for localized measurements, spaced among them with nonconducting fabric.

In case of socks only, therefore that do not have the function of interface with the insole, the socks can alternatively be realized in conducting fabric that is only internal. The entire sock could also be produced with standard filaments (cotton or synthetic) and the conductive patches placed or woven only internally.

In all said cases, except for that in which they serve as interface, the socks are furnished with electric wires to allow the connection to an external device of measurement.

Further advantages can be deduced from the other dependent claims.

### Brief description of drawings

Further features and advantages of the present device, as per the invention, will result to be clearer with the description that follows of some preferred embodiments, made to illustrate but not to limit, with reference to the annexed drawings, wherein:
- Figure 1 shows a pair of general plantars, while figure 2 shows the placement of a foot on a plantar;
- Figure 3 shows the application of conductive plates to the plantars, as per the present invention;
- Figures from 4 to 8 show two possible forms of realization for the present invention;
- Figure 9 and figure 10 show schematically the way with which such insoles allow to infer one or more physiological parameters;
- Figure 11 shows examples of socks provided with electrodes.

### Description of some preferred embodiments

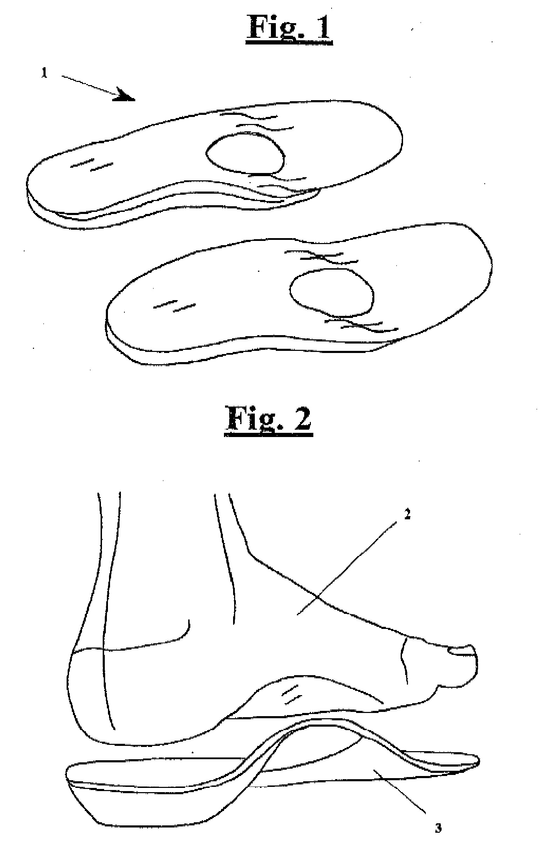

Figure 1 shows as a way of example two plantars (or insoles) as per the state of the art.

The plantars are supports for the foot, or simply separators between the sole of the shoe and the foot, which can be inserted into the shoe.

They trace the size and the shape of the foot that thus finds a correct and comfortable support, as for example shown in figure 2 (above all in case of pathologies in which the foot has an incorrect support).

The plantars are well known in the state of the art and can be available in multiple shapes and sizes, which are not the specific object of the present invention (see figures 1 and 2).

With reference to the subsequent figures from 3 to 10 the possible solutions are instead highlighted as per the present invention.

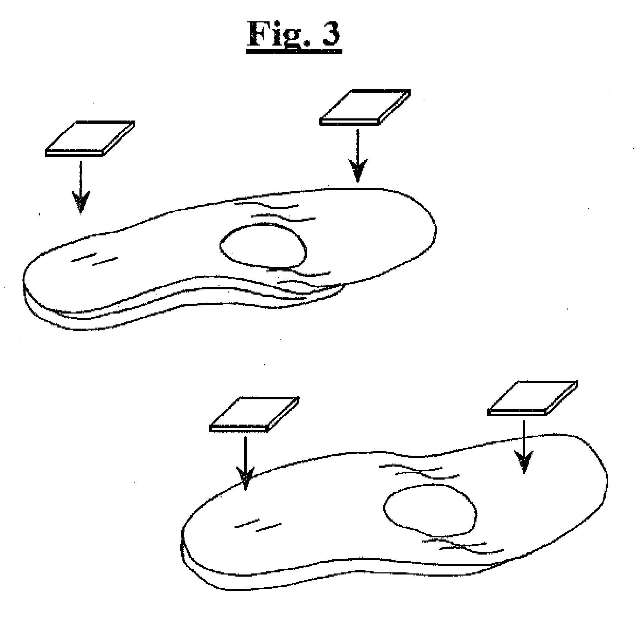

In accordance with figure 3, each plantar is furnished with conductive plates so as to make a measurement in accordance with the tetra-polar technique through an injection of current.

The possible solutions can be of two types.

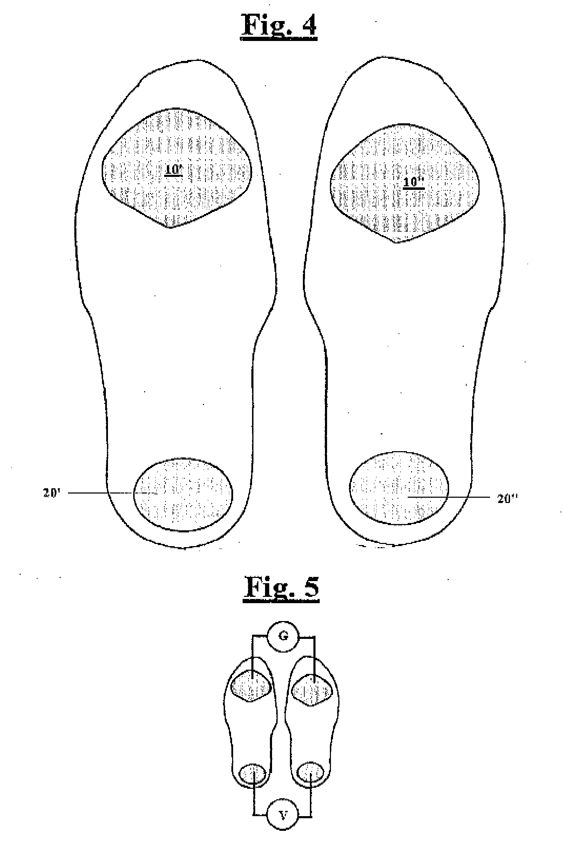

The first one is shown in figure 4 and in figure 5.

In this case two injecting electrodes (10', 10") are foreseen arranged one for each insole, whose injecting electrodes, as per figure 5, are connected to a generator (G). The receiving electrodes (20', 20") (always one for each insole) are instead connected to a voltmeter (V) that measures the voltage drop consequent to the injection of current in the points of the body of contact with the plates 10' and 10".

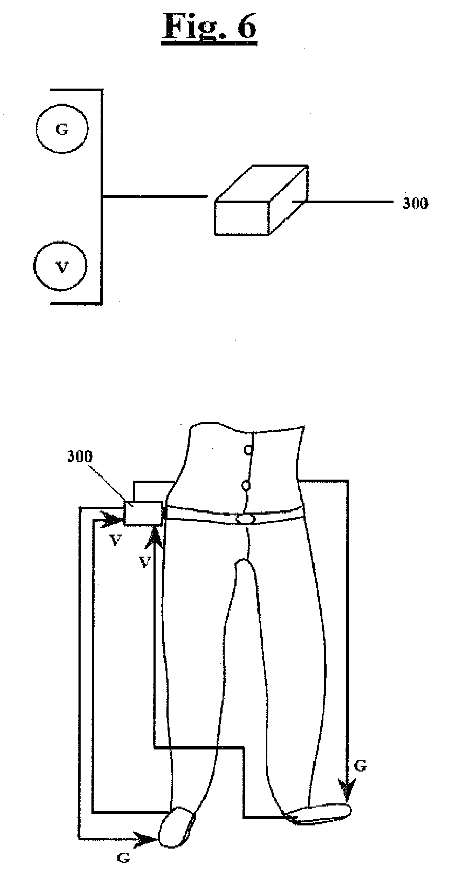

In such an embodiment, generator and voltmeter are preferably lodged in a small box 300 to be fastened to the waist of the user (as schematized in figure 6). They connect to the respective plates (see figure 5) through external wires schematized also them in figure 6.

In addition, further, the generator and the voltmeter are capable of communicating in wireless mode (for example with the Bluetooth technique) to an external device (for example, a mobile telephony device such as an I-Phone or Smartphone, a Tablet, a PC and the like). Such devices are obviously furnished with an appropriate processor that can process/memorize/visualize the data received.

It is enough to operate an easy and appropriate conditioning and digitalization of the signal to realize such a communication.

The external processor receives the data (or the value of voltage drop and/or dephasement) to make the measurements.

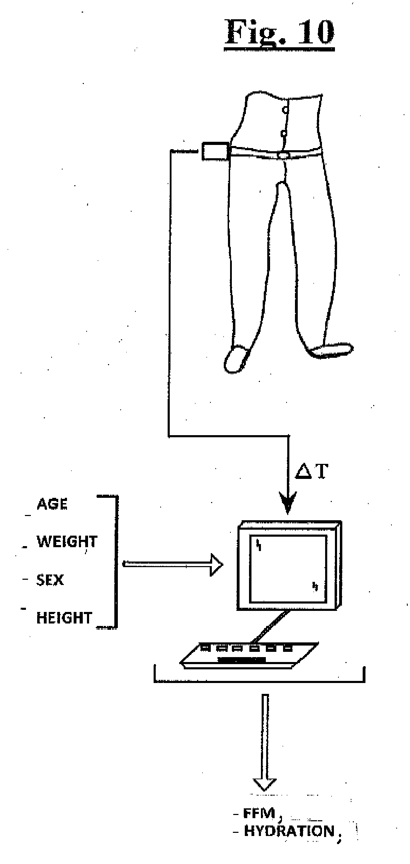

This is, for example, schematized in figure 10 in which the voltage drop read is sent to the external processor, preferably in wireless mode, whose processor processes such a datum with formulas that are well known and tested to obtain one or more physiological parameters.

This system is functional since it is possible to programme the processor (P) so that it requires the insertion of further personal data of the subject, for example age, sex, height, weight, etc. The system of figure 6 and relative figure 10 measures in fact a voltage drop in the inferior hemibody (inferior part of the body) and this results to be very useful in the medical field for a calculation, for example, of the thin mass, apart from the hydration condition, thanks to well-known formulas that implement said data inserted in the initial phase of processing.

Figure 6 schematizes said box with the inlets of the wires and the elements contained inside it. The box can be fastened to a waist and figure 6 shows schematically the directions of the wires that connect from the generator (G) to the relative plates and from the voltmeter (V) always to the relative plate.

There exist formulas known for some time to calculate physiological parameters such as the body composition cited above with foot-foot measurements obtained. They are, as said, considered sufficiently precise since they are already used on impedentiometric scales.

For example, we indicate the following Internet address:
http://www.nutritionj.com/content/14/1/52
from where the following formula for the estimation of the thin mass can be found:
(FFM-13.055+0.204 weight+0.394 height²/Impedance - 0.136 age + 8.125 sex (sex: Female = 0, Male = 1), r² = 0.92, standard error of the estimate = 3.17 kg).

This embodiment of the invention is particularly useful since it allows to obtain precise and repetitive measurements on subjects that need consecutive observations (monitoring).

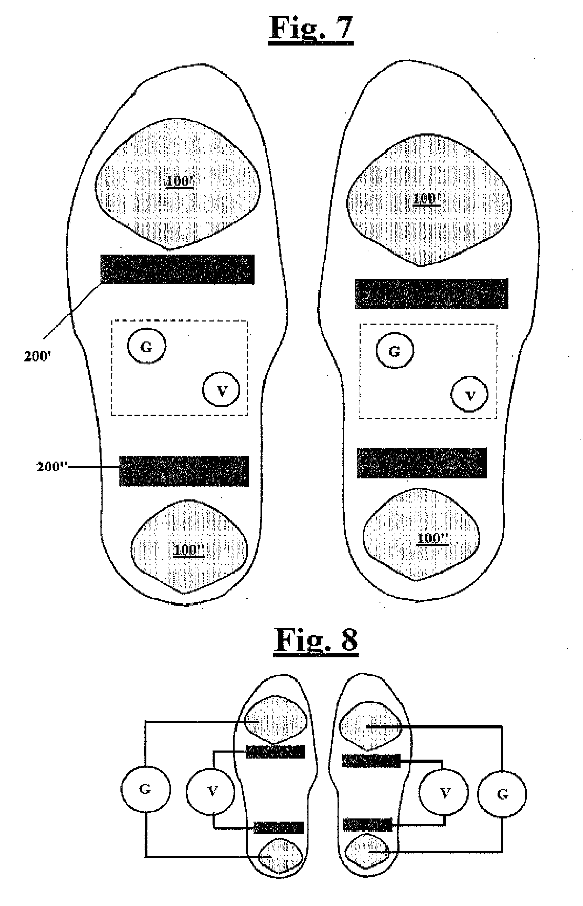

A variant of the invention is shown in figure 7, 8 and 9, which is particularly suitable for monitoring athletes during the motor activity, with particular attention to the rise of possible alterations of tissue or to study the evolution thereof.

The preceding embodiment, in fact, is not indicated for sporting use since it is rather cumbersome due to the external wires.

The solution that will now be described is much more compact and therefore insertable in shoes for sporting use. Further, as clarified below, being its range of action localized in the plantar area of the foot and not on the entire inferior hemibody, as per the preceding solution, it allows to make health condition checks focused on the foot itself (for example, the appearance of fasciitis, blisters, callosities, trauma ecchymosis, skin injuries, infections, etc.).

Going further into the descriptive detail of such a further configuration, in this case each plantar foresees a pair of injecting plates (or the plates 100', 100") and a pair of receiving plates (or the plates 200', 200").

In this way, the circulating current and the relative voltage drop and phase are measured on a more reduced segment and referred to the plantar area of the foot.

Generator and voltmeter are installed into the body of each insole and in the body of each shoe and connected with specific connectors to the electrodes of the insoles. Figure 7, in fact, schematizes also the presence of generator G and voltmeter V, connected to microprocessor for the processing and transmission of the data.

In accordance with such a tetra-polar technique, limiting the measurement to the section of the foot, various physiological parameters can be calculated, among which the condition of the tissues for diagnosing or preventing, for example, the fasciitis of the foot.

In this way, a football player can be monitored during a training session to verify the integrity of the tissues or other.

The injuries cause, in fact, an alteration of the circulation of current injected, therefore a curve delimiting an area of normality experimentally speaking can be created, for example analyzing a sample of healthy subjects or disposing of basic initial data that serve for the self-control.

The processor memorizes such graphics and verifies if the voltage drop measured in the specific subject falls within the graphics or not to verify if we are in a normality area or not.

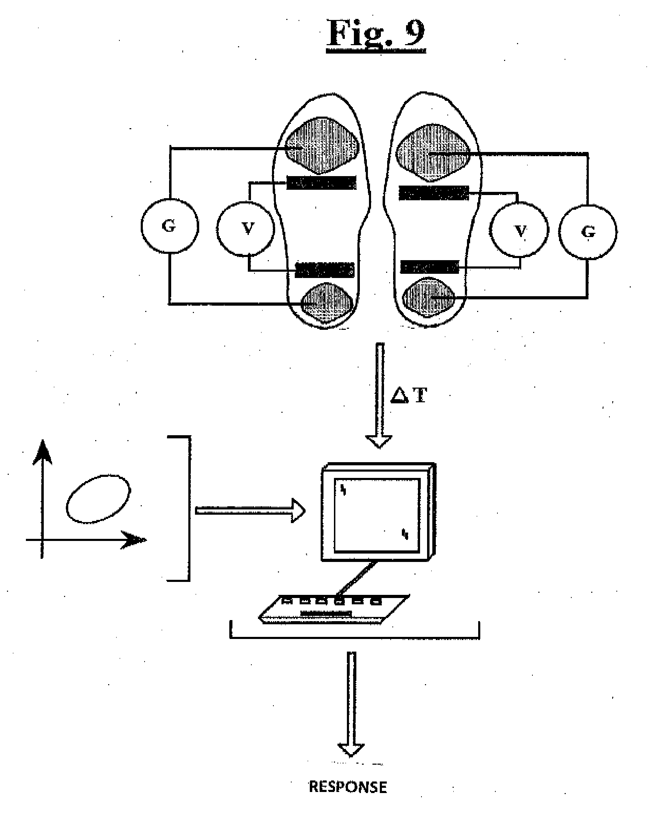

Likewise, figure 9 schematizes the functioning of this embodiment.

The voltage drop at various frequencies, or the resistance and reactance, are sent, preferably in wireless mode, to an external processor (for example an I-Phone that the athlete, the marathon runner, can wear). The processor acquires in a continuative manner the datum and verifies if it falls within the parameters of normality or not and every time memorizes or transmits such data in wireless mode so that they can be consulted by the expert medical staff.

Above all, in such an embodiment, but also in the preceding one, it is necessary to have a feeding that lasts in time.

All the systems described are therefore furnished with integrated feeding, in the form of rechargeable batteries, for example.

In any case, it can be thought of using recharging systems that are well known in the state of the art and that allow to produce current with the motion.

One of these is, for example, the well-known piezoelectric system that associates well with the compression of motion of the pace of the athlete.

A super-condenser can be integrated, above all, capable of storing big quantities of current to send at need to the battery or foresee simple batteries that are directly charged by such kinematic recharging systems, such as for example a mechanism similar to that of wrist watches.

The present invention is addressed to the creation of insoles of any nature and use integrating such a technology, since in this way they allow an insertion in any type of shoe.

While the technology described in figure 4 and 5 requires necessarily the use of a pair of insoles, the technology of figure 7, if wanted, can be implemented using a single insole, for example in case the pathology of a single foot wants to be monitored.

Further, nothing would exclude the realization of shoes integrating such a technology.

The use of the shoes is almost always accompanied by the use of socks.

For example, the specific solution highlighted in figure 7, which has been indicated in the present text as useful to monitor the sportsmen during their sporting activity, can require the use of a sock in the sense that the sportsman that has to run for a long time almost always uses specific socks to avoid the formation of blisters and to favour the absorption of the perspiration and the stability of the foot.

Naturally, the use of a normal sock in fabric creates a barrier of contact between skin and electrodes of the insole, altering the functioning thereof.

Substantially, the motor activity is hardly executed on bare foot, and therefore the electric detection in the presence of non-conductive socks would be impossible.

Having said all that, the system, consequently, foresees the presence of socks furnished with conductive sections woven in correspondence of the electrodes placed in the plantars, with the function of conductive interface that allows the passage of the electric current.

In particular, it is enough to realize a sock which is in part sewn with a yarn that is highly conductive and that therefore serves as interface between foot and insole.

The electrically conductive fabrics and suitable for such an application are well known and are therefore not described in further detail here.

The sock has to have the parts that trace the electrodes present in the insole realized in a conducting yarn and so as to fall within the area of such electrodes.

For example, with reference to the solution of figure 7, but the same is also valid also for the solution of figure 4 with two electrodes, it will be enough to weave socks that have such conducting yarns as to trace the shape and the position of the electrodes. The conductive parts of the socks are preferably of circular shape, because it is known that such an embodiment allows a more homogeneous transmission of the impedentiometric signals, but anyway with such sizes as to fall entirely within the area of the electrodes of the insole.

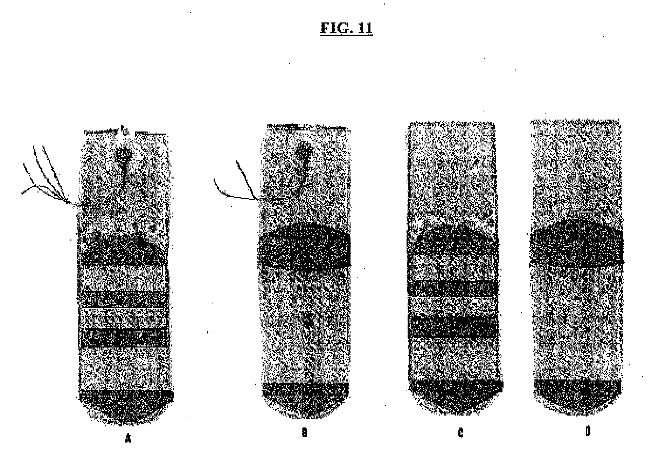

Figure 11 shows, in the example C and D, the case of socks in which the electrodes are shown that trace the electrodes of the insole and that serve as simple interface between foot and what is present in the insole. The electrodes obtained in the sock thanks to the use of conducting yarn are isolated among them through the rest of the realization material of the sock, not conducting. Basically, having said the above, the current injected passes from the electrode of the insole to the sock and from here to the foot. In the inverse sense, the voltage drop is detected.

In this easy way, the user is free to wear socks, avoiding the formation of sores and blisters.

Nothing would impede to realize normal socks furnished with holes in correspondence of the position of the electrodes to then place and fix regular electrodes, avoiding the sewing of the socks in part with regular fabric and in part with conducting fabric.

A variant of the invention is shown in figure 11 with the sock A and B. This solution differs from the preceding C and D in the fact that, as shown in figure 11, the electric wires are foreseen that from the electrodes of the sock depart towards the outside and are configured to connect to an external electronics (generator, voltmeter, processor). Such electronics can be that present in the insole of figure 7 or the external box 300 of figure 6 like also an external machinery in itself furnished with generator, voltmeter and processor.

The advantage of this solution, in particular of the solution A of figure 11, is that which allows to monitor the advancement of pathologies, as described, on subjects immobilized on the bed. In this way, such patients can be monitored by inserting the sock when they are in bed and connecting the sock to an external measurement device, for example an external BIA.

This can be useful in patients having bedsores and also a single sock can be useful if it is necessary to monitor a single foot.

The solution A and B of figure 11, foreseeing specific wires that connect to an external device, unlike those that can have the function of interface with the insole, allow a realization of conductive fabric also only internal (this is because it is enough that the skin has contact with the electrode without the wish of interface with an external electrode present in an insole). In that case, the entire sock could be produced with standard filaments (cotton or synthetic ones) and the conductive "patches" placed or woven only internally.

Also in this case the use of two electrodes allows a measurement of hemibodies, while the solution with four electrodes (embodiment C) allows localized measurements.

## Claims

1. A footwear system **characterized in that** it comprises at least one injecting electrode (10', 10", 100', 100"), through which to inject a current (I) of reference, and at least one receiving electrode (20', 20"; 200', 200") through which to measure a relative voltage drop (V) and/or a variation of phase.

2. A footwear system, as per claim 1, wherein said footwear system is at choice in the form of:
- A single insole;
- A pair of insoles;
- A single sock (A-B-C-D);
- A pair of socks (A-B-C-D)

3. A footwear system, as per claim 1 or 2, wherein a pair of injecting electrodes (100', 100") and a pair of receiving electrodes (200', 200") are foreseen.

4. A footwear system, as per one or more of the preceding claims from 1 to 3, wherein a generator (G) connected to the injecting electrode/s (10', 10", 100', 100") and a voltmeter (V) connected to the receiving electrode/s (20', 20"; 200', 200") are foreseen.

5. A footwear system, as per one or more of the preceding claims from 1 to 4, wherein, in case of a pair of insoles, a single injecting electrode (10', 10") and a single receiving electrode (20', 20") for each insole of said pair are foreseen.

6. A footwear system, as per one or more of the preceding claims, wherein the generator (G) and the voltmeter (V) are placed inside a containment box (300) that is outside of the insoles.

7. A footwear system, as per one or more of the preceding claims from 1 to 4, wherein, in case of a pair of insoles, a pair of injecting electrodes (10', 10") and a pair of receiving electrodes (20', 20") for each insole of said pair are foreseen.

8. A footwear system, as per one or more of the preceding claims, wherein the generator (G) and the voltmeter (V) are placed inside the body of each insole.

9. A footwear system, as per one or more of the preceding claims, wherein in case of a single insole, a pair of injecting electrodes (10', 10") and a pair of receiving electrodes (20', 20") are foreseen.

10. A footwear system, as per one or more of the preceding claims, wherein, in case of single insole or pair, a sock (A-B-C-D), or a pair of socks, is further foreseen, having at least one of its conducting parts arranged so as to overlap in use to the electrodes present in the insole or in the pair of insoles.

11. A footwear system, as per one or more of the preceding claims, wherein, in case of sock (A-B-C-D), said sock foresees at least one injecting electrode, preferably two injecting electrodes, for injecting a current of reference (I) and at least one receiving electrode, preferably two receiving electrodes, for measuring a voltage drop (V), and relative electric wires connected to the electrodes and that allow to connect said electrodes to an external device so as to inject through said external device said current of reference and measure a relative voltage drop.

12. A footwear system, as per claim 11, wherein the electrodes of said sock are in the form of a conducting yarn that reproduces the electrode or in the form of an electrodes applied internally to the body of the sock.

13. A footwear system, as per one or more of the preceding claims, wherein an external processor (P) is foreseen, for example a PC, a portable computer, a mobile telephony device, and communicating, preferably in wireless mode, with said footwear system.

14. A footwear system, as per claim 13, wherein the processor (P) is programmed to calculate/memorize one or more physiological parameters, eventually on the basis of one or more parameters indicative of the subject under exam and loadable in the processor.

15. A footwear system, as per one or more of the preceding claims, wherein an integrated feeding system is foreseen, wherein, preferably, said feeding system foresees a rechargeable system of the kinematic type, for example of the piezoelectric or accelerometer type, and wherein, still more preferably, a super-condenser connected to the rechargeable system is further foreseen.
